# EUROPÄISCHE PATENTANMELDUNG

(11) **EP 1 350 513 A1**
(43) Veröffentlichungstag der Anmeldung: **08.10.2003**
(21) Anmeldenummer: 02007647.7
(22) Anmeldetag: 04.04.2002
(51) Int. Cl.: A61K 31/565, A61K 9/48

(54) **Weichgelatinekapsel enthaltend ein 17-alpha-estradiol und Ascorbinsäure**

(71) Anmelder: Jenapharm GmbH & Co. KG, 07745 Jena (DE)
(72) Erfinder: Fricke, Dr. Sabine, 07749 Jena (DE); Pfeifer, Manuela, 07745 Jena (DE); Papantoniou, Ioannis, 45659 Recklinghausen (DE); Zielke, Petra, 07745 Jena (DE)
(74) Vertreter: Störle, Christian, Dr.

(57) **Zusammenfassung**

Beschrieben werden Weichgelatinekapseln, die als Wirkstoff ein 17α-Estradiol und als Stabilisator dafür Ascorbinsäure enthalten. Ferner bezieht sich die Anmeldung auf die Verwendung von Ascorbinsäure zur Stabilisierung von 17α-Estradiolen.

## Beschreibung

Die Erfindung bezieht sich auf eine Weichgelatinekapsel, die ein stabilisiertes Steroid enthält, so wie die Verwendung von Ascorbinsäure zur Stabilisierung spezieller Steroide in Weichgelatinekapseln.

Aus der DE 42 39 945 A1 sind 14α, 15α-Methylen-Steroide der Formel (1) bekannt 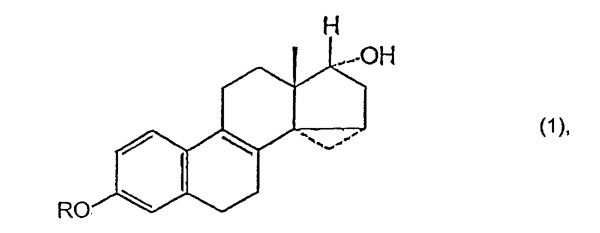 in der R für H oder eine C₁-C₄-Alkyl-Gruppe steht.
Diese Steroide weisen eine fertilitätshemmende Wirkung auf und werden deshalb als Wirkstoffe in Arzneimitteln eingesetzt.

Allerdings sind diese Steroide in kristalliner Form bei Raumtemperatur instabil. Als Hauptzersetzungsprodukt bildet sich β-Methylendiol. Somit konnten herkömmliche pharmazeutische Formulierungen, wie Tabletten und Gelatinesteckkapseln, für vorgenannte Steroide nicht eingesetzt werden.

Der vorliegenden Erfindung liegt somit die Aufgabe zu Grunde, eine pharmazeutische Formulierung bereitzustellen, in der insbesondere vorgenannte Steroide stabilisiert sind.

Erfindungsgemäß wird dies durch ein Weichgelatinekapsel erreicht, die als Wirkstoff ein 17α-Estradiol und als Stabilisator dafür Ascorbinsäure enthält.

Unter dem Ausdruck "ein 17α-Estradiol" werden Verbindungen jeglicher Art verstanden, die als Grundkörper das Estra-1,3,5(10)-trien-3,17α-diol aufweisen. Bei diesem Grundkörper kann 1 oder mehrere, z.B. 2, der H-Atome durch organische Reste, wie C₁-C₄-Alkyl-, C₁-C₄-Alkenyl-, C₁-C₄-Alkinyl- oder OH-Rest, oder zwei benachbarte H-Atome des Grundkörpers durch - CH₂-substituiert sein. Des weiteren können zwei benachbarte C-Atome des Grundkörpers, z.B. die C-Atome mit den Nummern 8 und 9, durch eine Doppelbindung verbunden sein.

Vorzugsweise weist das 17α-Estradiol die Formel (1) auf

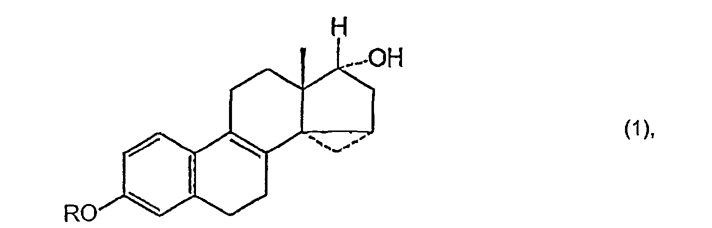

in der R für H oder ein C₁-C₄-Alkyl steht. Insbesondere ist das in der erfindungsgemäßen Weichgelatinekapsel eingesetzte 17α-Estradiol 14α, 15α-Methylen-estra-1,3,5(10),8-tetraen-3,17α-diol, das als besonders instabiles aber sehr gut wirksames Steroid in günstiger Weise sehr effektiv mit Ascorbinsäure stabilisiert werden kann.

Die Erfindung beruht auf der Erkenntnis, daß die vorgenannten Steroide in Weichgelatinekapseln mit Ascorbinsäure besonders gut stabilisiert werden. Die Steroide werden dabei mit Ascorbinsäure in Lösung gebracht und in einer Weichgelatinekapsel verkapselt. Es wurde vollkommen überraschend festgestellt, daß andere Stabilisatoren, wie Butylhydroxyanisol, Butylhydroxytoluol oder Ascorbylpalmitat, hinsichtlich ihrer stabilisierenden Wirkung kaum oder deutlich weniger effektiv als Ascorbinsäure waren, so daß sie die Steroide nicht so stabilisieren konnten, daß eine Lagerung von Arzneimitteln während eines ausreichend langen Zeitraums, beispielsweise über zwei bis drei Jahre, möglich ist.

Weichgelatinekapseln weisen eine Hülle auf, die einen Innenraum umschließt. Als Hüllenmaterial kann Gelatine oder succinylierte Gelatine eingesetzt werden. Sowohl die Herstellung von Weichgelatinekapseln als auch der Hülle ist dem Fachmann bekannt. Er kennt hierzu notwendige Materialien und Verfahrensschritte.

Neben dem Vorteil, daß der Wirkstoff in einem flüssigen Medium in Lösung gebracht werden kann, wodurch der Wirkungseintritt schnell erfolgt, bietet die Weichgelatinekapsel die Vorteile einer Dosen-Einheitsform. Zusätzlich wird wegen der Kapselhülle ein hoher Widerstand gegen Oxidation und Lichtabbau erreicht, so daß sie zur Stabilisierung des Steroids beiträgt.

Im von der Hülle umschlossenen Innenraum befindet sich als Wirkstoff ein 17α-Estradiol zusammen mit der Ascorbinsäure in gelöster oder suspendierter Form. Wirkstoff und Ascorbinsäure können in einem Lösungsmittel gelöst sein, das z.B. Propylenglycol und/oder mittelkettige Partialglyceride umfaßt. Falls es erforderlich ist, kann ein Lösungsvermittler, z.B. Macrogolglycerol-hydroxystearat, zugesetzt werden.

Die erfindungsgemäßen Weichgelatinekapseln können neben dem vorgenannten Steroid noch weitere Wirkstoffe enthalten, beispielsweise Dehydroepiandrosteron (DHEA) und/oder dessen Sulfat-Ester (DHEAS), sofern eine Gabe zusammen mit vorgenanntem Steroid indiziert ist.

Je nach Dosierung des Wirkstoffs kann die Kapselgröße zwischen 5 minims/oval oder oblong und 14 minims/oblong bzw. 10 minims/oval liegen.

Für die Herstellung des wirkstoffhaltigen Kapselfüllguts der Weichgelatinekapseln kommen zwei Varianten in Betracht, nämlich die mit hydrophilem und die mit hydrophobem Füllgut. Bei der Variante mit hydrophilem Füllgut wird das Steroid zusammen mit der Ascorbinsäure in einer Mischung von Lösungsmittel und/oder Lösungsvermittler, z.B. mittelkettige Partialglyceriden, Propylenglycol und Macrogolglycerolhydroxystearat, gelöst. Danach wird ggf. der weifere Wirkstoff, wie DHEA und/oder DHEAS zugesetzt. Je nach Dosierung insbesondere von DHEA entsteht eine Lösung oder eine Suspension. Im Falle einer Suspension kann zur Viskositätserhöhung ein üblicher (hydrophiler) Matrixbildner, beispielsweise makromolekulare Schleimstoffe, wie Hydroxyethylcellulose, Natriumcarboxymethylcellulose oder PEG, in den pharmazeutisch üblichen Konzentrationen zugesetzt werden. Bei der Variante mit hydrophobem Füllgut wird als Grundlage dafür ein mittelkettiges Triglycerid verwendet, das in eine ethanolische Lösung des Steroids und der Ascorbinsäure, eingetragen wird. Zu dieser Mischung wird dann ggf. der weitere Wirkstoff, z.B. DHEA und/oder DHEAS, unter Rühren fein eingetragen. Es entsteht eine Suspension, die durch pharmazeutisch übliche hydrophobe Matrixbildner, beispielsweise Aerolsil oder Aluminiumstearat, noch verstärkt werden kann. Die so hergestellten Lösungen bzw. Suspensionen können nach dem bekannten Scherer-Verfahren zu Weichgelatinekapseln verarbeitet werden. Dabei kann sowohl die übliche Standardgelatine als auch succinylierte Gelatine als Hüllenmaterial zum Einsatz kommen.

Die Menge des als Wirkstoff eingesetzten Steroids in der Weichgelatinekapsel kann in Abhängigkeit von der zu behandelnden Erkrankung und der Häufigkeit der Verabreichung des Medikaments gewählt werden.

Da während der Lagerdauer die Ascorbinsäure durch Stabilisierungsreaktionen verbraucht wird, wird die Ascorbinsäuremenge in der Weichgelatinekapsel so gewählt, daß sie für die Lagerungsdauer ausreicht. Allerdings sollte die Menge nicht so groß sein, daß die Ascorbinsäure eine Vernetzung der Gelatine bewirkt. Unter Berücksichtigung vorstehender Überlegungen hat sich gezeigt, daß das Steroid vorzugsweise in einer Menge von 0,005 Gew.-% bis 4 Gew.-%, bezogen auf die Kapselfüllmasse, und die Ascorbinsäure vorzugsweise in einer Menge von 0,025 Gew.-% bis 4 Gew.-%, bezogen auf die Kapselfüllmasse, vorliegt.

Eine besonders günstige Zusammensetzung der Weichgelatinekapsel kann folgende Bestandteile aufweisen: Die Kapselhülle wird in üblicher Weise unter Verwendung von Gelatine oder succinylierter Gelatine hergestellt. Die Zusammensetzung des Kapselinhalts kann bei einer Gesamtmasse des Inhalts von 250 mg bis 1000 mg das Steroid in einer Menge von 0,05 bis 10 mg, die Ascorbinsäure in einer Menge von 0,25 mg bis 10 mg, Lösungsmittel, wie Propylenglycol und/oder mittelkettige Partialglyceride in einer Menge von 25 mg bis 688 mg, beispielsweise 25 mg bis 100 mg Propylenglycol und/oder 151,7 mg bis 588 mg mittelkettige Partialglyceride, und Lösungsvermittler, beispielsweise Macrogolglycerolhydroxystearat, in einer Menge von 73 mg bis 292 mg. Die Menge des weiteren Wirkstoffs, z.B. DHEA und/oder DHEAS, kann 10 mg bis 200 mg betragen.

Gegenstand der vorliegenden Erfindung ist ferner die Verwendung von Ascorbinsäure zur Stabilisierung eines 17α-Estradiols, insbesondere in Weichgelatinekapseln. Hinsichtlich des Steroids und der Zusammensetzung der Weichgelatinekapsel wird auf vorstehende Ausführungen verwiesen.

Die vorliegende Erfindung wird anhand der nachfolgenden Beispiele näher erläutert, ohne sie darauf einzuschränken.

### Beispiel 1: Herstellung von Weichgelatinekapseln enthaltend DHEA und 14α, 15α-Methylenestra-1,3,5(10),8-tetraen-3,17α-diol

Die Verbindung 14α, 15α-Methylenestra-1,3,5(10),8-tetraen-3,17α-diol wird in den Beispielen mit "Methylendiol-Steroid" bezeichnet.

### 1. hydrophiles Füllgut

| **Bestandteile** | **Zusammensetzung/Kapsel** |
|---|---|
| Methylendiol-Steroid | 0,2 mg |
| DHEA | 50,0 mg |
| Ascorbinsäure | 2,5 mg |
| Propylenglycol | 25,0 mg |
| Macrogolglycerol-hydroxystearat (Cremophor® RH 40) | 73,0 mg |
| Mittelkettige Partialglyceride (Imwitor® 742) | 149,3 mg |
| Gesamtmasse 300,0 mg | |

### 2. hydrophiles Füllgut mit Suspensionsverstärker

| **Bestandteile** | **Zusammensetzung/Kapsel** |
|---|---|
| Methylendiol-Steroid | 0,2 mg |
| DHEA | 50,0 mg |
| Ascorbinsäure | 2,5 mg |
| Propylenglycol | 25,0 mg |
| Macrogolglycerol-hydroxystearat (Cremophor® RH 40) | 73,0 mg |
| Hydroxyethylcellulose | 7,5 mg |
| Mittelkettige Partialglyceride (Imwitor® 742) | 141,8 mg |
| Gesamtmasse 300,0 mg | |

### 3. hydrophobes Füllgut

| **Bestandteile** | **Zusammensetzung/Kapsel** |
|---|---|
| Methylendiol-Steroid | 0,2 mg |
| Ascorbinsäure | 2,5 mg |
| Ethanol | 10,0 mg |
| (DHEA) | 50,0 mg |
| Mittelkettige Triglyceride (Miglyol 812) | 37,3 mg |
| Gesamtmasse 100,0 mg | |

Es wurden in üblicher Weise Weichgelatinekapseln mit vorstehenden Zusammensetzungen unter Verwendung von Gelatine als Hüllenmaterial hergestellt.

### Beispiel 2: Untersuchungen der Stabilität von 14α, 15α-Methylenestra-1,3,5(10),8-tetraen-3,17α-diol (Methylendiol-Steroid) mit und ohne Ascorbinsäure

| | |
|---|---|
| **Darreichungsform** | Weichgelatinekapsel |
| **Farbe** | weiß |
| **Füllgewicht** | 250,00 mg +/- 3% |

**Tabelle 1:**

| **Rezeptur des Kapselinhalts von Methylendiol-Steroid-Weichgelatinekapseln** | | | |
|---|---|---|---|
| **Methylen-Steroid** | **Wirkstoff** | **0,2 mg / Kapsel 1** | **5 mg / Kapsel 2** |
| Ascorbinsäure | Stabilisator | 2,50 mg | 2, 50 mg |
| Proplenglycol | Lösungsmittel | 25,00 mg | 25,00 mg |
| Macrogolglycerolhydroxystearat (Cremophor RH 40) | Lösungsvermittler | 73,00 mg | 73,00 mg |
| Mittelkettige Partialglyceride (Imwitor 742) | Lösungsmittel | 149,30 mg | 144,50 mg |

Als Hüllenmaterial wurde Gelatine verwendet. Die Herstellung der Weichgelatinekapsel erfolgt in üblicher Weise.

**Tabelle 2:**

| **Stabilität von Methylendiol-Steroid (25°C/60 % rel. Feuchte)** | | | | | |
|---|---|---|---|---|---|
| Dargestellt ist der Vergleich der Zunahme des Hauptzersetzungsproduktes β-Methylendiol in reinem Wirkstoff und Methylendiol-Steroid-Weichgelatinekapseln, die mit Ascorbinsäure stabilisiert wurden (Tabelle 1). | | | | | |

| **Gehalt β-Methylendiol in (%)** | | | | | |
|---|---|---|---|---|---|
| | **Start** | **2 Monate** | **3 Monate** | **6 Monate** | **12 Monate** |
| Methylendiol-Steroid (reiner Wirkstoff *) | 0,5 | 1,6 | 1,7 | | |
| Methylendiol-Steroid (reiner Wirkstoff *) | 0,4 | 1,7 | 1,5 | | |
| Methylendiol-Steroid (reiner Wirkstoff *) | 0,1 | 1,2 | 1,5 | | |
| Kapsel 1 | 0,29 | - | 0,57 | 0,43 | 0,74 |
| Kapsel 2 | 0,38 | - | 0,56 | 0,58 | 0,70 |

| | | | | | |
|---|---|---|---|---|---|
| *) Es wurden 3 Chargen eingesetzt. | | | | | |

**Tabelle 3:**

| **Stabilität von Methylendiol-Steroid bei 5°C und ungeregelter Luftfeuchtigkeit** | | | | | |
|---|---|---|---|---|---|
| Dargestellt ist der Vergleich der Zunahme des Hauptzersetzungsproduktes β-Methylendiol in reinem Wirkstoff und Methylendiol-Steroid-Weichgelatinekapseln, die mit Ascorbinsäure stabilisiert wurden (Tabelle 1). | | | | | |

| **Gehalt β-Methylendiol in (%)** | | | | | |
|---|---|---|---|---|---|
| | **Start** | **2 Monate** | **3 Monate** | **6 Monate** | **12 Monate** |
| Methylendiol-Steroid (reiner Wirkstoff **) | 0,5 | 0,5 | 0,4 | 0,6/1,1*) | 1,5 |
| Methylendiol-Steroid (reiner Wirkstoff **) | 0,4 | 0,5 | 0,6 | 1,3/1,0*) | 1,2 |
| Methylendiol-Steroid (reiner Wirkstoff **) | 0,1 | 0,2 | 0,1 | 0,4/0,6*) | 1,9 |
| Kapsel 1 | 0,29 | - | 0,54 | 0,47 | 0,73 |
| Kapsel 1 | 0,38 | - | 0,50 | 0,58 | 0,70 |

| | | | | | |
|---|---|---|---|---|---|
| *) Änderung der Spezifikation, deshalb Doppelbestimmung nach beiden Spezifikationen | | | | | |
| **) Es wurden 3 Chargen eingesetzt. | | | | | |

## Patentansprüche

1. Weichgelatinekapsel, enthaltend als Wirkstoff ein 17α-Estradiol und als Stabilisator dafür Ascorbinsäure.

2. Kapsel nach Anspruch 1, wobei das 17α-Estradiol die Formel (1) aufweist 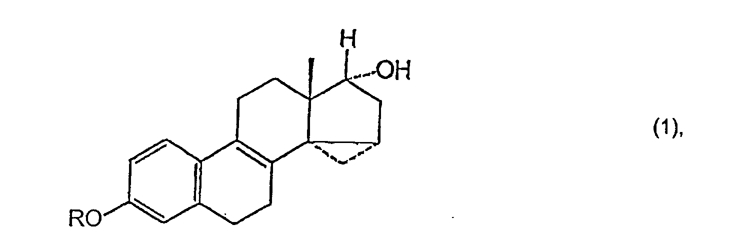 in der R für H oder ein C₁-C₄-Alkyl steht.

3. Kapsel nach Anspruch 2, wobei die Verbindung der Formel (1) 14α,15α-Methylen-estra-1,3,5(10),8-tetraen-3,17α-diol ist.

4. Kapsel nach einem der vorhergehenden Ansprüche, wobei das Steroid in einer Menge von 0,005 Gew.-% bis 4 Gew.-%, bezogen auf die Kapselfüllmasse, vorliegt.

5. Kapsel nach einem der vorhergehenden Ansprüche, wobei die Ascorbinsäure in einer Menge von 0,025 Gew.-% bis 4 Gew.-%, bezogen auf die Kapselfüllmasse, vorliegt.

6. Verwendung von Ascorbinsäure zur Stabilisierung von 17α-Estradiol.
